## Europäisches Patentamt
## European Patent Office
## Office européen des brevets

(19)

(11) Veröffentlichungsnummer: **0 011 090**
**B1**

(12)

# EUROPÄISCHE PATENTSCHRIFT

(45) Veröffentlichungstag der Patentschrift:
**27.01.82**

(51) Int. Cl.³: **C 07 C 85/11,** C 07 C 87/52,
B 01 J 23/64

(21) Anmeldenummer: **79102930.9**

(22) Anmeldetag: **13.08.79**

(54) **Verfahren zur katalytischen Hydrierung von Nitrobenzol.**

(30) Priorität: **11.11.78 DE 2849002**

(43) Veröffentlichungstag der Anmeldung:
**28.05.80 Patentblatt 80/11**

(45) Bekanntmachung des Hinweises auf die Patenterteilung:
**27.01.82 Patentblatt 82/4**

(84) Benannte Vertragsstaaten:
**AT BE DE FR GB IT NL SE**

(56) Entgegenhaltungen:
**DE-A-2 214 056**
**DE-A-2 255 498**
**GB-B-1 356 400**
**US-A-3 830 847**

(73) Patentinhaber: **BAYER AG, Zentralbereich Patente, Marken und Lizenzen, D-5090 Leverkusen 1, Bayerwerk (DE)**

(72) Erfinder: **Birkenstock, Udo, Dr., Schneppersdelle 2, D-4030 Ratingen 8 (DE)**
Erfinder: **Lachmann, Burkhard, Dr., Lettweg 3, D-4005 Meerbusch 1 (DE)**
Erfinder: **Metten, Josef, Dr., Deswatinesstrasse 24, D-4150 Krefeld (DE)**
Erfinder: **Schmidt, Herbert, Im Hederichsfeld 52, D-5090 Leverkusen 3 (DE)**

# Verfahren zur katalytischen Hydrierung von Nitrobenzol

Die vorliegende Erfindung betrifft ein Verfahren zur katalytischen Hydrierung von Nitrobenzol zu Anilin in der Gasphase unter Verwendung eines palladiumhaltigen Trägerkatalysators.

Palladiumhaltige Trägerkatalysatoren zur Durchführung katalytischer Hydrierungen, u. a. auch von Nitroaromaten, sind allgemein bekannt. Die hierbei in der Technik verwendeten Träger als Ausgangsbasis für die entsprechenden Trägerkatalysatoren sind jedoch mit erheblichen Nachteilen behaftet. So sind die Trägermaterialien aufgrund ihrer Oberflächenbeschaffenheit nicht inert und bedingen daher in der Regel die Bildung unerwünschter Nebenprodukte. Weiterhin führt die Aufbringung des Palladiums auf diese Träger im allgemeinen zu einer gleichmäßigen Pd-Verteilung über das gesamte Trägerkorn. Dies führt zu unnötig hohen Katalysatorkosten, da bekanntlich die im Inneren des Trägerkorns abgeschiedenen Wirksubstanzen entweder gar nicht oder nur in untergeordnetem Maße an der zu katalysierenden Reaktion teilnehmen. Beispielsweise werden Aluminiumspinelle, insbesondere Alkali- oder Erdalkalispinelle, als Trägermaterialien für Palladiumkatalysatoren zur Reduktion von Nitroaromaten eingesetzt. Bei der Herstellung solcher Alkali- oder Erdalkali-Aluminium-Spinelle bleiben verfahrensbedingt unterschiedliche Restmengen wasserlöslichen Alkalis bzw. Erdalkalis im Trägerkorn verteilt. Durch diese Inhomogenität des Spinells ergibt sich zwangsläufig eine inhomogene Abscheidung der Wirksubstanzen in den einzelnen Trägerkörnern. Als Folge davon treten beim technischen Einsatz dieser Aluminiumspinell-Katalysatoren unerwünschte Aktivitäts-, Selektivitäts- und Standzeitunterschiede auf (DE-OS 2 135 155).

Da die katalytische Hydrierung von Nitrobenzol zu Anilin ein großtechnisches Verfahren darstellt und Anilin als wichtiges Zwischenprodukt bei der Herstellung von Pharmazeutika, Farbstoffen und von Monomeren für die Polymerisation in großen Mengen benötigt wird, besteht weiterhin ein verstärktes Interesse daran, dieses Verfahren durch Eliminierung der obengenannten Nachteile zu verbessern.

Aus der DE-OS 2 214 056 ist ein Palladium-haltiger Katalysator für die Reduktion von Nitroverbindungen bekannt, der Palladium zusammen mit Vanadin und/oder Vanadinverbindungen auf Aluminium-Spinellen als Träger enthält. Das Trägermaterial kann dabei innere Oberflächen im Bereich 20 bis 120 m²/g aufweisen. Diese Literaturstelle enthält keinerlei Hinweise darauf, daß Trägermaterialien mit einer inneren Oberfläche von unter 20 m²/g dann besonders vorteilhaft sind, wenn sie vor der Aufbringung der katalytisch wirksamen Komponenten mit einer Base behandelt und anschließend in bestimmter Weise getrocknet werden.

Es wurde nun ein Verfahren zur Hydrierung von Nitrobenzol zu Anilin in Gegenwart von ein Edelmetall enthaltenden Trägerkatalysatoren in der Gasphase bei erhöhter Temperatur gefunden, bei dem man die Hydrierung in Gegenwart eines Mehrkomponenten-Trägerkatalysators durchführt, der 1 bis 20 g eines Edelmetalls pro Liter Trägermaterial und 1 bis 20 g eines oder mehrerer Übergangsmetalle der Gruppen IVa, Va oder VIa des Periodensystems der Elemente (Mendelejew) pro Liter Trägermaterial elementar oder in Form einer Verbindung sowie gegebenenfalls 1 bis 20 g eines weiteren Übergangsgruppen- oder Hauptgruppenelements pro Liter Trägermaterial elementar oder in Form einer Verbindung enthält und wobei der inerte Träger eine kleine innere Oberfläche aufweist und das dadurch gekennzeichnet ist, daß der inerte Träger eine BET-Oberfläche von weniger als 20 m²/g aufweist und, vor dem in an sich bekannter Weise erfolgenden Aufbringen der katalytisch wirksamen Komponenten, mit einer Base vorbehandelt und anschließend auf einen Restfeuchtegehalt von weniger als 10% der maximalen Saugfähigkeit des Trägers getrocknet worden ist.

Bevorzugt verwendet man im erfindungsgemäßen Verfahren Dreikomponentenkatalysatoren, auf denen 1 bis 10 g Edelmetall pro Liter Trägermaterial und 5 bis 15 g eines oder mehrerer Übergangsmetalle, vorzugsweise Vanadin, Niob, Tantal, Chrom, Molybdän, Wolfram oder Titan pro Liter Trägermaterial elementar oder in Form einer beliebigen Verbindung, vorzugsweise einer sauerstoffhaltigen Verbindung, sowie gegebenenfalls 1 bis 10 g eines weiteren Übergangsgruppen- oder Hauptgruppenelements, vorzugsweise eines der Elemente Blei, Zink oder Wismut pro Liter Trägermaterial elementar oder in Form einer beliebigen Verbindung abgeschieden sind, wobei als inertes Trägermaterial beispielsweise $\alpha$-Al$_2$O$_3$ mit einer BET-Oberfläche von weniger als 20 m²/g, bevorzugt weniger als 10 m²/g dient, das durch Einwirkung einer Base und anschließende Trocknung auf eine Restfeuchte von bevorzugt weniger als 5%, besonders bevorzugt weniger als 2%, und ganz besonders bevorzugt weniger als 1% der Saugfähigkeit des Trägers vorbehandelt wird, bevor man es mit der Edelmetallsalzlösung, vorzugsweise Palladiumsalzlösung, und mit einer oder gegebenenfalls mehreren Zusatzkomponenten, z. B. Vanadin-, Blei- oder Wismutsalzen, tränkt.

Unter dem Begriff Edelmetalle werden Metalle der Gruppen VIIIa, Ib und IIb des Periodensystems nach Mendelejew verstanden. Im einzelnen seien beispielsweise genannt Fe, Ru, Os, Co, Rh, Ir, Ni, Pd, Pt, Cu, Ag, Au, Zn, Cd, Hg. Besonders bevorzugt wird als Edelmetall Palladium eingesetzt. Als Hauptgruppenelement sei ein Metall der Gruppen IVb und Vb, z. B. Blei oder Wismut, genannt.

Das erfindungsgemäße Verfahren kann in allen für die Gasphasenhydrierung geeigneten Reaktortypen, wie z. B. Röhrenreaktoren, Wirbelbettreaktoren oder Käfigreaktoren betrieben werden. Bevorzugt wird das erfindungsgemäße Verfahren in Röhrenreaktoren mit fest angeordnetem Katalysator durchgeführt. Es kann sowohl drucklos als auch bei Überdruck oder vermindertem Druck gearbeitet werden. Im allgemeinen wird die Reaktion annähernd drucklos unter den an sich bekannten Temperaturbedingungen für die katalytische Nitroaromaten-Hydrierung betrieben. Die Reaktionswärme wird mit einer geeigneten Wärmeträgerflüssigkeit, deren Temperatur in einem Bereich von etwa 150 bis 350°C, bevorzugt 200 bis 300°C, gehalten wird, abgeführt.

Das Nitrobenzol wird vor Durchströmen des Katalysatorbettes in einem Verdampfer im Wasserstoffstrom mit mindestens 3 Mol Wasserstoff, bevorzugt 4 bis 10 Mol Wasserstoff pro Mol Nitrobenzol bei einem Temperaturbereich von etwa 100 bis 300°C, bevorzugt 150 bis 250°C, verdampft. Der überschüssige Wasserstoff wird gewöhnlich unter Ausschleusen eines geringen Teilstromes im Kreislauf geführt. Nach Durchströmen des Reaktors wird das Hydrierprodukt kondensiert.

Als Trägermaterialien, die mit Basen nach dem erfindungsgemäßen Verfahren vorbehandelt werden, kommen die verschiedensten Systeme, aus denen das inerte Trägermaterial mit einer BET-Oberfläche von kleiner als 20 m²/g, bevorzugt kleiner als 10 m²/g, aufgebaut ist, zur Anwendung. Es handelt sich hierbei im wesentlichen um Metalloxide, Silikate, Spinelle, Carbide, Carbonate usw. und Mischungen derselben. Besonders bevorzugt sind inerte Trägermaterialien wie z. B. Aluminiumoxide, Siliciumdioxide, Siliciumdioxid-Aluminiumoxid-Gemische, amorphe Kieselsäure, Kieselgure, Barium-, Strontium- oder Calciumcarbonate, Mischungen derselben, gegebenenfalls unter Zusatz von Siliciumdioxiden oder Aluminiumoxiden, Titanoxide, Zirkonoxide, Magnesiumoxide, Magnesiumsilikate, Zirkonsilikate, Magnesium-Aluminium-Spinell, Siliciumcarbide, Wolframcarbide, Mischungen von Siliciumcarbiden mit Siliciumdioxiden oder beliebige Mischungen derselben. Die inerten Träger können in den verschiedensten Formen zur Anwendung kommen, wie z. B. als Kugeln, Granulate, Extrudate, Tabletten, Sattelkörper, Rohrabschnitte, Bruchstücke, Wabenkeramik usw.

Die Besonderheiten in der Herstellung für die in dem erfindungsgemäßen Verfahren verwendeten Trägerkatalysatoren werden im folgenden kurz beschrieben:

Die Behandlung des inerten als Trägermaterial besonders bevorzugten $\alpha$-Al$_2$O$_3$-Trägers mit einer Base erfolgt normalerweise bei Temperaturen von 10 bis 60°C. Es werden bevorzugt 0,5 bis 20 Grammäquivalente Base pro Grammäquivalente Palladium eingesetzt. Bevorzugte Basen sind Alkalihydroxide, Erdalkalihydroxide, Alkalicarbonate, Alkalihydrogencarbonate und Alkaliacetate. Sie werden üblicherweise als Lösungen in Wasser oder in nichtwäßrigen Lösungsmitteln, wie aliphatischen Alkoholen mit 1 bis 4 C-Atomen oder aliphatischen Ketonen, wie z. B. Aceton oder in Mischungen derselben eingesetzt. Die besonders bevorzugten Basen sind Natriumhydroxid und Kaliumhydroxid.

Wesentlich ist die an die Basenbehandlung sich anschließende Trocknung des Trägermaterials bis auf den angegebenen Restfeuchtegehalt. Sie erfolgt im allgemeinen bei 50 bis 200°C, bevorzugt bei 100 bis 150°C, und bei Drücken von ≤1 bar.

Anschließend wird der basevorbehandelte Träger entsprechend seiner Saugfähigkeit mit einer Palladiumsalzlösung, deren Pd-Gehalt durch die vorher aufgebrachte Basemenge festgelegt ist, in an sich bekannter Weise bis zur Sättigung getränkt. Gegebenenfalls wird das aufgebrachte Pd-Salz zum Metall reduziert, bevor im nächsten Herstellungsschritt eines oder mehrere der Übergangsmetalle der Gruppen IVa, Va oder VIa des Periodensystems nach Mendelejew, z. B. V, Cr, Mo, W, Nb, Ta, Ti, in Form einer beliebigen Metallsalzlösung, beispielsweise als Metalloxalatlösung, aufgebracht werden. Es kann auch vorteilhaft sein, die Auftränkung der Edelmetallsalzlösung gleichzeitig mit der Übergangsmetallsalztränkung durchzuführen, beispielsweise Pd gemeinsam mit Cr in Form ihrer Chloride aufzubringen. Nach Trocknung und anschließender Zersetzung des Metallsalzes bei etwa 200 bis 500°C, bevorzugt 250 bis 400°C, wird das Trägermaterial gegebenenfalls in einem weiteren Herstellungsschritt mit der Salzlösung eines Übergangs- oder Hauptgruppenelements der Gruppen IIb, IVb und Vb, bevorzugt mit einer Blei- und/oder Wismutsalzlösung, getränkt.

Wesentlich für die Herstellung der in dem erfindungsgemäßen Verfahren verwendeten Trägerkatalysatoren ist, daß durch die Art der Basevorbehandlung des Trägers und die nachgeschaltete Trocknung die Wirkstoffabscheidung, insbesondere die Edelmetallabscheidung in einer schmalen, äußeren Ringzone kurz unterhalb der Oberfläche des Trägers erfolgt. Die nach diesem Prinzip hergestellten und in dem erfindungsgemäßen Verfahren verwendeten Katalysatoren liefern bei ihrem technischen Einsatz in der Nitrobenzolhydrierung aufgrund der niedrigen Oberfläche des inerten Trägermaterials weniger Nebenprodukte als herkömmliche Hydrierkatalysatoren. Weiterhin sind die im Träger angereicherten Wirksubstanzen weitgehend vor Vergiftungen und Verlusten durch Abrieb geschützt. Hierdurch wird eine sehr hohe Lebensdauer der Katalysatoren bei gleichbleibender Aktivität erreicht.

Als Katalysatoren, die in dem erfindungsgemäßen Verfahren zur katalytischen Hydrierung von Nitrobenzol zu Anilin in der Gasphase Verwendung finden, seien beispielsweise erwähnt:

0 011 090

Tabelle 1

| Katalysator | Pd (g/l) | V (g/l) | Mo (g/l) | Cr (g/l) | Pb (g/l) | Bi (g/l) | Zn (g/l) |
|---|---|---|---|---|---|---|---|
| I | 9 | 9 | – | – | 3 | – | – |
| II | 9 | 6 | – | – | 3 | – | – |
| III | 6 | 9 | – | – | 3 | – | – |
| IV | 4,5 | 6 | – | – | 3 | – | – |
| V | 9 | 9 | – | – | – | – | – |
| VI | 9 | 12 | – | – | 3 | – | – |
| VII | 9 | 9 | – | – | – | 3 | – |
| VIII | 9 | – | 6 | – | 3 | – | – |
| IX | 9 | – | – | 7,2 | – | – | – |
| X | 9 | 12 | – | – | – | – | – |
| XI | 9 | 9 | – | – | – | – | 3 |

Die nachfolgenden Beispiele dienen der weiteren Erläuterung des erfindungsgemäßen Verfahrens.

## Beispiel 1

### Katalysatorherstellung

Ein Liter eines $\alpha$-Aluminiumoxidträgers in Kugelform mit 3 bis 6 Millimeter Durchmesser, einer BET-Oberfläche von 9,8 $m^2/g$, einer Saugfähigkeit von 45,1 ml Wasser pro 100 g Träger und einem Schüttgewicht von 812 g/l wurde mit 366 ml einer 10,8 g, entsprechend 0,27 Grammäquivalenten, NaOH enthaltenden wäßrigen Lösung imprägniert. Die Lösung wurde innerhalb weniger Minuten völlig vom Träger aufgenommen. Der feuchte Träger wurde in ein senkrechtes Glasrohr von ca. 2 l Inhalt geschüttet und im Warmluftstrom von 120° C bei einer Luftmenge von 25 $Nm^3$ Luft pro Stunde getrocknet. Die Trockenzeit bis zur Gewichtskonstanz betrug etwa 30 Minuten. Der Restfeuchtegehalt lag nach dem Abkühlen auf Raumtemperatur bei ca. 0,9% der Saugfähigkeit des Trägers.

Der so vorbehandelte trockene Träger wurde seiner Saugfähigkeit entsprechend mit 366 ml einer wäßrigen Natriumtetrachloropalladat-(II)-Lösung, die 9 g Palladium, entsprechend 0,169 Grammäquivalent, enthielt, getränkt und 15 Minuten stehen gelassen. Zur Reduktion der auf dem Träger abgeschiedenen Palladiumverbindung zu metallischen Palladium wurde der Träger in einem Becherglas mit 400 ml einer 10%igen wäßrigen Hydrazinhydrat-Lösung überschichtet und 2 Stunden stehen gelassen. Danach wurde der Katalysator in einem fließenden Strom von destilliertem Wasser gewaschen, bis keine Ionen der bei der Katalysatorherstellung verwendeten Verbindungen im Waschwasser mehr nachweisbar waren, was nach 10 Stunden der Fall war.

Die anschließende Trocknung wurde im Warmluftstrom vorgenommen wie vorher bei der Trocknung des Trägers beschrieben. Der so hergestellte Katalysator enthielt 9 g Palladium pro Liter Träger.

Der Pd-haltige Katalysator wurde anschließend mit 366 ml einer wäßrigen, 9 g Vanadium in Form von Vanadyloxalat enthaltenden Lösung getränkt. Die Trocknung des mit Vanadyloxalat imprägnierten Pd-Katalysators erfolgte analog der Trocknung des Trägers im Warmluftstrom bei 120° C. Die anschließende Zersetzung des Vanadyloxalates wurde während 6 Stunden bei 300° C durchgeführt.

Nach dieser Behandlung wurde der Katalysator mit 366 ml einer wäßrigen, 3 g Blei in Form von Bleiacetat enthaltenden Lösung getränkt. Der mit Bleiacetat imprägnierte Katalysator wurde in feuchtem Zustand in einen Rohrreaktor gefüllt und während der Aufheizperiode des Wärmeträgers getrocknet.

Die Aktivierung des Katalysators wurde im Wasserstoffstrom bei der Temperatur des Wärmeträgers, die 280° C betrug, durchgeführt. Der fertige Katalysator enthielt, als Metall berechnet, 9 g Palladium, 9 g Vanadium und 3 g Blei pro Liter Träger. Er entspricht dem Katalysator I in Tabelle 1.

4

## Beispiel 2

Ein Rohrreaktor (55 Rohre; Rohrdurchmesser 25 mm; Rohrlänge 3500 mm) wurde mit 85 Liter des nach Beispiel 1 hergestellten Katalysators I (s. Tabelle) befüllt. Durch den mit der Wärmeträgerflüssigkeit »Marlotherm S« auf 280°C vorgeheizten Reaktor wurde von unten nach oben ein gasförmiges Nitrobenzol-Wasserstoff-Gemisch mit einem Mol-Verhältnis von 1 : 6 geleitet. Die Belastung betrug 0,47 kg Nitrobenzol pro Stunde pro Liter Kontakt. Nach 1450 Stunden war die Reaktionszone von unten nach oben durchwandert, und es wurde regeneriert. In den folgenden Fahrperioden lagen die einzelnen Laufzeiten zwischen 1020 h und 1550 h. Mit der Katalysatorfüllung wurde eine Gesamtstandzeit von 7850 Stunden erreicht. Die dabei produzierte Anilinmenge betrug 236 Tonnen. Daraus errechnet sich ein Katalysatorverbrauch von 0,36 Liter Katalysator pro Tonne Anilin. Die auf den theoretischen Nitrobenzolumsatz bezogene Anilinausbeute lag bei 99,86 Gew.-%.

## Beispiel 3

Ein Einrohr-Hydrierreaktor (Rohrdurchmesser 32 mm; Rohrlänge 1300 mm) wurde mit 1 Liter des Katalysators II der Tabelle 1 befüllt. Die Herstellung des Katalysators erfolgte analog Beispiel 1, wobei jedoch 6 g Vanadium in Form von Vanadyloxalat aufgebracht wurden. Die Reaktortemperatur wurde durch siedendes Diphyl als Wärmeträger bei 260 bis 270°C gehalten. Das vorher im Wasserstoffstrom bei 160 bis 180°C verdampfte Nitrobenzol durchströmte als gasförmiges Nitrobenzol-Wasserstoff-Gemisch (Mol-Verhältnis 1 : 6) den Reaktor von unten nach oben. Die Belastung wurde bei 0,5 kg Nitrobenzol/h.l Kontakt gehalten. Nach Verlassen des Reaktors wurde das gasförmige Reaktionsprodukt kondensiert. Die Laufzeiten der einzelnen Fahrperioden lagen zwischen 900 und 1600 h. Die Gesamtstandzeit betrug 8000 h. Dabei wurden 3030 kg Anilin produziert. Das entspricht einem Katalysatorverbrauch von 0,33 l Katalysator/t Anilin. Die über die Gesamtstandzeit gemittelte Anilinausbeute lag bei 99,85% der Theorie.

## Beispiel 4

Die katalytische Gasphasenhydrierung von Nitrobenzol zu Anilin wurde in einem Einrohr-Hydrierreaktor analog Beispiel 3 mit dem Katalysator III der Tabelle 1 durchgeführt. Die Herstellung dieses Katalysators erfolgte analog Beispiel 1. In Abweichung zu den dortigen Angaben wurde jedoch mit 366 ml einer 7,2 g NaOH (entsprechend 0,18 Grammäquivalent) enthaltenden wäßrigen Lösung imprägniert. Die Palladiumtränkung erfolgte mit 366 ml einer wäßrigen Natriumtetrachloropalladat-(V)-Lösung, die 6 g Palladium, entsprechend 0,112 Grammäquivalent, enthielt.

Die bei der Nitrobenzolhydrierung mit diesem Katalysator erzielten Laufzeiten der einzelnen Fahrperioden lagen zwischen 1100 h und 1360 h.

Ergebnisse:

| | |
|---|---|
| Gesamtstandzeit: | 6140 h |
| Katalysatorverbrauch: | 0,43 l/t Anilin |
| Anilinausbeute | 99,81 Gew.-% der Theorie |

## Beispiel 5

Es wurde analog Beispiel 3 mit dem Katalysator IV der Tabelle 1 hydriert. Die Herstellung des Katalysators erfolgte analog Beispiel 1, wobei jedoch mit 5,4 g, entsprechend 0,135 Grammäquivalenten, NaOH imprägniert wurde. Die Palladiumtränkung erfolgte mit 4,5 g entsprechend 0,084 Grammäquivalenten Palladium und für die Vanadiumabscheidung wurden 6 g Vanadium in Form von Vanadyloxalat eingesetzt. Bei der Nitrobenzolhydrierung mit diesem Katalysator lagen die Laufzeiten der einzelnen Fahrperioden zwischen 1000 h und 1430 h.

Ergebnisse:

| | |
|---|---|
| Gesamtstandzeit: | 7650 h |
| Katalysatorverbrauch: | 0,35 l/t Anilin |
| Anilinausbeute: | 99,87 Gew.-% der Theorie |

## Beispiel 6

Analog Beispiel 3 wurde der Katalysator X der Tabelle 1 zur Hydrierung von Nitrobenzol eingesetzt. Die Herstellung erfolgte analog Beispiel 1, wobei jedoch 12 g Vanadium in Form von 366 ml einer wäßrigen Vanadyloxalatlösung aufgebracht wurden und die anschließende Bleiimprägnierung entfiel.

Ergebnisse:

| | |
|---|---|
| Gesamtstandzeit: | 6450 h |
| Katalysatorverbrauch: | 0,41 h/t Anilin |
| Anilinausbeute: | 99,82 Gew.-% der Theorie |

## Beispiel 7

Analog Beispiel 3 wurde der Katalysator XI der Tabelle 1 zur Hydrierung von Nitrobenzol eingesetzt. Seine Herstellung erfolgte analog Beispiel 1, wobei jedoch die Bleitränkung entfiel und statt dessen 3 g Zink in Form von 366 ml einer wäßrigen Zinkacetatlösung aufgebracht wurden.

Ergebnisse:

| | |
|---|---|
| Gesamtstandzeit: | 7120 h |
| Katalysatorverbrauch: | 0,37 l/t Anilin |
| Anilinausbeute: | 99,84 Gew.-% der Theorie |

## Patentanspruch

Verfahren zur Hydrierung von Nitrobenzol zu Anilin in Gegenwart von ein Edelmetall enthaltenden Trägerkatalysatoren in der Gasphase bei erhöhter Temperatur, wobei man die Hydrierung in Gegenwart eines Mehrkomponenten-Trägerkatalysators durchführt, der 1 bis 20 g eines Edelmetalls pro Liter Trägermaterial und 1 bis 20 g eines oder mehrerer Übergangsmetalle der Gruppen IVa, Va oder VIa des Periodensystems der Elemente (Mendelejew) pro Liter Trägermaterial elementar oder in Form einer Verbindung sowie gegebenenfalls 1 bis 20 g eines weiteren Übergangsgruppen- oder Hauptgruppenelements pro Liter Trägermaterial elementar oder in Form einer Verbindung enthält und wobei der inerte Träger eine kleine innere Oberfläche aufweist, dadurch gekennzeichnet, daß der inerte Träger eine BET-Oberfläche von weniger als 20 m$^2$/g aufweist und, vor dem in an sich bekannter Weise erfolgenden Aufbringen der katalytisch wirksamen Komponenten, mit einer Base vorbehandelt und anschließend auf einen Restfeuchtegehalt von weniger als 10% der maximalen Saugfähigkeit des Trägers getrocknet worden ist.

## Claim

Process for the hydrogenation of nitrobenzene to aniline, in the presence of supported catalysts containing a noble metal, in the gas phase at elevated temperature, wherein the hydrogenation is carried out in the presence of a multi-component supported catalyst which contains 1 to 20 g of a noble metal per litre of support material and 1 to 20 g of one or more transition metals of groups IVa, Va or VIa of the periodic table of the elements (Mendeleev) per litre of support material, the said metals being in the form of the elements or in the form of a compound, and also, optionally, 1 to 20 g of a further transition group element or main group element, in the form of the elements or in the form of a compound, per litre of support material, the inert support having a small inner surface area, characterised in that the inert support has a BET surface area of less than 20 m$^2$/g and, prior to application of the catalytically active components, which is effected in a manner which is in itself known, has been pre-treated with a base and then dried to a residual moisture content of less than 10% of the maximum absorbency of the support.

## Revendication

Procédé d'hydrogénation du nitrobenzène en aniline en présence de catalyseurs supportés contenant un métal noble, en phase gazeuse et à température élevée, dans lequel l'hydrogénation est exécutée en présence d'un catalyseur supporté à plusieurs composants qui contient 1 à 20 g d'un métal noble par litre de matériau de support et 1 à 20 g d'un ou de plusieurs métaux de transition des groupes IVa, Va VIa du système périodique des éléments (Mendeleīev) par litre de matériau de support, à l'état élémentaire ou sous forme d'un composé, de même qu'éventuellement 1 à 20 g d'un autre élément des groupes de transition ou des groupes principaux par litre de matériau de support, à l'état élémentaire ou sous forme d'un composé, et dans lequel le support inerte présente une petite surface interne, caractérisé en ce que le support inerte présente une surface BET inférieure à 20 m²/g et en ce qu'avant l'application qui a lieu de manière connue en elle-même des composants catalytiquement actifs, il est prétraité avec une base, puis il est séché à une teneur résiduelle en humidité de moins de 10% de la capacité maximale d'absorption du support.